# EUROPEAN PATENT SPECIFICATION

(11) **EP 3 084 446 B1**
(45) Date of publication and mention of the grant of the patent: **14.12.2022**
(21) Application number: 14872420.6
(22) Date of filing: 19.12.2014
(51) Int. Cl.: G01N 33/68, G01N 33/574, G01N 33/573, A61K 31/50, C12Q 1/68

(54) **HISTONE DEACETYLASE 6 (HDAC6) BIOMARKERS IN MULTIPLE MYELOMA**
HISTONDEACETYLASE 6 (HDAC6)-BIOMARKER BEI MULTIPLEM MYELOM
BIOMARQUEURS DE L'HISTONE-DÉSACÉTYLASE 6 (HDAC6) DANS LE MYÉLOME MULTIPLE

(30) Priority: 20.12.2013 US 201361918934 P; 16.10.2014 US 201462064586 P
(43) Date of publication of application: 26.10.2016
(73) Proprietor: Acetylon Pharmaceuticals, Inc., Boston, MA 02210 (US)
(72) Inventor: YANG, Min, Newton Center, MA 02459 (US); TAMANG, David, Lee, Watertown, MA 02472 (US); JONES,Simon, S., Harvard, MA 01451 (US)
(74) Representative: D Young & Co LLP
(86) International application number: PCT/US2014/071348
(87) International publication number: WO 2015/095632

(56) References cited:
- WO-A1-2009/080437
- WO-A1-2010/139812
- WO-A1-2012/087983
- WO-A1-2014/197471
- WO-A2-2013/033627
- Anonymous: "Discovery Histone Deacetylase (HDAC)6 Specific Proteomic Biomarkers In Multiple Myeloma (MM) Using Stable Isotope Labeling By Amino Acids In Cell Culture (SILAC) | Blood Journal", , 1 January 2013 (2013-01-01), XP055361281, Retrieved from the Internet: URL:http://www.bloodjournal.org/content/12 2/21/1909?sso-checked=true [retrieved on 2017-04-03]
- Min Yang ET AL: "Discovery of Histone Deacetylase (HDAC)6 Specific Proteomic Biomarkers in Multiple Myeloma (MM) using Stable Isotope Labeling by Amino acids in Cell Culture (SILAC) Acknowledgements", , 6 December 2013 (2013-12-06), XP055361574, Retrieved from the Internet: URL:http://www.acetylon.com/docs/poster_AS H_Poster-SILAC-Min.pdf [retrieved on 2017-04-04]
- Anonymous: "GEO Accession viewer", , 18 December 2012 (2012-12-18), XP055361608, Retrieved from the Internet: URL:https://www.ncbi.nlm.nih.gov/geo/query /acc.cgi?acc=GPL16382 [retrieved on 2017-04-04]
- C I JONES ET AL: "Identification of circulating microRNAs as diagnostic biomarkers for use in multiple myeloma", BRITISH JOURNAL OF CANCER, vol. 107, no. 12, 20 November 2012 (2012-11-20), pages 1987-1996, XP055361624, GB ISSN: 0007-0920, DOI: 10.1038/bjc.2012.525
- WALKINSHAW D.R. ET AL.: 'Histone deacetylase inhibitors as novel anticancer therapeutics.' CURRENT ONCOLOGY vol. 15, no. 5, 2008, pages 70 - 76, XP055353541
- RHODES L.V. ET AL.: 'The histone deacetylase inhibitor trichostatin A alters microRNA expression profiles in apoptosis-resistant breast cancer cells' ONCOLOGY REPORTS. vol. 27, no. 1, 2012, pages 10 - 16, XP055353554

## Description

### FIELD OF THE INVENTION

Provided herein are methods for monitoring the treatment efficiency of a histone deacetylase 6 (HDAC6) inhibitor in a subject.

### BACKGROUND OF THE INVENTION

Cancer is one of the leading causes of death in the United States and in the world.

Cancer grows out of normal cells in the body. Normal cells multiply when the body needs them, and die when the body doesn't need them. Cancer occurs when the cells in the body grow and multiply out of control.

There are many causes of cancer, such as exposure to carcinogenic chemicals, use of tobacco, drinking excess alcohol, exposure to environmental toxins, exposure to excessive sunlight, genetic problems, obesity, radiation, and viruses. In addition, the cause of many cancers remains unknown.

There are many different types of cancer, which can develop in almost any organ or tissue in the body. One type of cancer is multiple myeloma.

Multiple myeloma, also known as plasma cell myeloma or Kahler's disease, is a cancer of plasma cells. In multiple myeloma, collections of abnormal plasma cells accumulate in the bone marrow, where they interfere with the production of normal blood cells.

Because many organs can be affected by myeloma, the symptoms and signs vary greatly. Effects of myeloma include elevated calcium, renal failure, anemia, and bone lesions.

Myeloma is generally thought to be treatable, but incurable. Remission may be induced with steroids, chemotherapy, proteasome inhibitors, immunomodulatory drugs such as thalidomide or lenalidomide, and stem cell transplants.

Myeloma develops in 1-4 per 100,000 people per year. With conventional treatment, median survival is 3-4 years, which may be extended to 5-7 years or longer with advanced treatments. Multiple myeloma is the second most common hematological malignancy in the U.S. (after non-Hodgkin lymphoma), and constitutes 1% of all cancers.

Accordingly, there is a need to quickly and reliably identify biomarkers that are indicative of treatment efficiency in multiple myeloma.

### SUMMARY OF THE INVENTION

The present invention is described in the appended claims.

The invention provides a method for monitoring the treatment efficiency of a histone deacetylase 6 (HDAC6) inhibitor in a subject comprising: a) providing a biological sample having been taken from a subject who has received a therapeutically effective amount of an HDAC6 inhibitor; b) determining the amount of the HDAC6 biomarker RNA (ribonucleic acid) consisting of the nucleic acid sequence of SEQ ID NO: 26 in the sample; and c) concluding that the HDAC6 treatment is efficient if the HDAC6 biomarker RNA consisting of the nucleic acid sequence of SEQ ID NO: 26 is down-regulated; wherein the HDAC6 inhibitor is Compound A 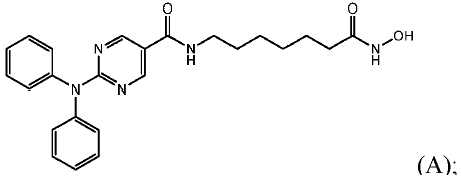 or Compound B

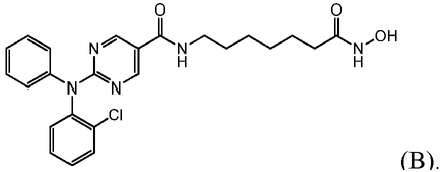

In specific embodiments, the sample is a myeloma sample. In other specific embodiments, the sample is a bone marrow sample.

In specific embodiments, step c) comprises concluding that the HDAC6 treatment is efficient if the HDAC6 biomarker RNA consisting of the nucleic acid sequence of SEQ ID NO: 26 is down-regulated by 2-fold or more.

In specific embodiments, the HDAC6 inhibitor is Compound A. In other specific embodiments, the HDAC6 inhibitor is Compound B.

### DETAILED DESCRIPTION OF THE INVENTION

### Definitions

Unless defined otherwise, all technical and scientific terms used herein have the same meaning as is commonly understood by one of ordinary skill in the art.

The articles "a" and "an" are used herein to refer to one or more than one (*i.e.,* to at least one) of the grammatical object of the article. By way of example, "a biomarker" means one biomarker or more than one biomarker.

The term "about" generally indicates a possible variation of no more than 10%, 5%, or 1% of a value. For example, "about 25 mg/kg" will generally indicate, in its broadest sense, a value of 22.5-27.5 mg/kg, i.e., 25 ± 2.5 mg/kg.

The terms "administer" or "administration" refer to the act of injecting or otherwise physically delivering a substance as it exists outside the body (*e.g.,* in a formulation) into a patient, such as by mucosal, intradermal, intravenous, intramuscular delivery and/or any other method of physical delivery described herein or known in the art. When a disease, or a symptom thereof, is being treated, administration of the substance typically occurs after the onset of the disease or symptoms thereof. When a disease, or symptoms thereof, is being prevented, administration of the substance typically occurs before the onset of the disease or symptoms thereof.

The term "alkyl" refers to saturated, straight- or branched-chain hydrocarbon moieties containing, for example, between one and six, or one and eight carbon atoms, respectively. Examples of C₁₋₆ alkyl moieties include, but are not limited to, methyl, ethyl, propyl, isopropyl, *n*-butyl, *tert*-butyl, neopentyl, n-hexyl moieties; and examples of C₁₋₈ alkyl moieties include, but are not limited to, methyl, ethyl, propyl, isopropyl, *n*-butyl, *tert-butyl,* neopentyl, n-hexyl, heptyl, and octyl moieties.

The number of carbon atoms in an alkyl substituent can be indicated by the prefix "Cₓ-_{y}," where x is the minimum and y is the maximum number of carbon atoms in the substituent. Likewise, a Cₓ chain means an alkyl chain containing x carbon atoms.

The term "alkoxy" refers to an -O-alkyl moiety.

The terms "cycloalkyl" or "cycloalkylene" denote a monovalent group derived from a monocyclic or polycyclic saturated or partially unsaturated carbocyclic ring compound. Examples of C₃-C₈-cycloalkyl include, but are not limited to, cyclopropyl, cyclobutyl, cyclopentyl, cyclohexyl, cycloheptyl and cyclooctyl; and examples of C₃-C₁₂-cycloalkyl include, but are not limited to, cyclopropyl, cyclobutyl, cyclopentyl, cyclohexyl, bicyclo [2.2.1] heptyl, and bicyclo [2.2.2] octyl. Also contemplated are monovalent groups derived from a monocyclic or polycyclic carbocyclic ring compound having at least one carbon-carbon double bond by the removal of a single hydrogen atom. Examples of such groups include, but are not limited to, cyclopropenyl, cyclobutenyl, cyclopentenyl, cyclohexenyl, cycloheptenyl, cyclooctenyl, and the like.

The term "aryl" refers to a mono- or poly-cyclic carbocyclic ring system having one or more aromatic rings, fused or non-fused, including, but not limited to, phenyl, naphthyl, tetrahydronaphthyl, indanyl, idenyl and the like. Aryl groups can have 6 carbon atoms. Aryl groups can have from six to ten carbon atoms. Aryl groups can have from six to sixteen carbon atoms.

The term "heteroaryl" refers to a mono- or poly-cyclic (*e.g*., bi-, or tri-cyclic or more) fused or non-fused moiety or ring system having at least one aromatic ring, where one or more of the ring-forming atoms is a heteroatom such as oxygen, sulfur, or nitrogen. Heteroaryl groups can have from about one to six carbon atoms. Heteroaryl groups can have from one to fifteen carbon atoms. Heteroaryl groups can contain five to sixteen ring atoms of which one ring atom is selected from oxygen, sulfur, and nitrogen; zero, one, two, or three ring atoms are additional heteroatoms independently selected from oxygen, sulfur, and nitrogen; and the remaining ring atoms are carbon. Heteroaryl includes, but is not limited to, pyridinyl, pyrazinyl, pyrimidinyl, pyrrolyl, pyrazolyl, imidazolyl, thiazolyl, oxazolyl, isooxazolyl, thiazolyl, thiadiazolyl, oxadiazolyl, thiophenyl, furanyl, indolyl, quinolinyl, isoquinolinyl, benzimidazolyl, benzooxazolyl, quinoxalinyl, acridinyl, and the like.

The term "halo" refers to a halogen, such as fluorine, chlorine, bromine, and iodine.

The term "HDAC" refers to histone deacetylases, which are enzymes that remove the acetyl groups from the lysine residues in core histones, thus leading to the formation of a condensed and transcriptionally silenced chromatin. There are currently 18 known histone deacetylases, which are classified into four groups. Class I HDACs, which include HDAC1, HDAC2, HDAC3, and HDAC8, are related to the yeast RPD3 gene. Class II HDACs, which include HDAC4, HDAC5, HDAC6, HDAC7, HDAC9, and HDAC10, are related to the yeast Hda1 gene. Class III HDACs, which are also known as the sirtuins are related to the Sir2 gene and include SIRT1-7. Class IV HDACs, which contains only HDAC11, has features of both Class I and II HDACs. The term "HDAC" refers to any one or more of the 18 known histone deacetylases, unless otherwise specified.

The term "HDAC6 specific" means that the compound binds to HDAC6 to a substantially greater extent, such as 5X, 10X, 15X, 20X greater or more, than to any other type of HDAC enzyme, such as HDAC1 or HDAC2. That is, the compound is selective for HDAC6 over any other type of HDAC enzyme. For example, a compound that binds to HDAC6 with an IC₅₀ of 10 nM and to HDAC1 with an IC₅₀ of 50 nM is HDAC6 specific. On the other hand, a compound that binds to HDAC6 with an IC₅₀ of 50 nM and to HDAC1 with an IC₅₀ of 60 nM is not HDAC6 specific

The term "inhibitor" is synonymous with the term antagonist.

The term "biological sample" shall generally mean any biological sample obtained from an individual, body fluid, cell line, tissue culture, or other source. Body fluids are, for example, blood, lymph, sera, plasma, urine, semen, synovial fluid, and spinal fluid. Methods for obtaining tissue biopsies and body fluids from mammals are well known in the art. If the term "sample" is used alone, it shall still mean that the "sample" is a "biological sample", *i.e.,* the terms are used interchangeably.

The terms "composition" and "formulation" are intended to encompass a product containing the specified ingredients (*e.g*., an HDAC inhibitor) in, optionally, the specified amounts, as well as any product which results, directly or indirectly, from the combination of the specified ingredients in, optionally, the specified amounts.

The term "excipients" refers to inert substances that are commonly used as a diluent, vehicle, preservative, binder, stabilizing agent, etc. for drugs and includes, but is not limited to, proteins (*e.g.,* serum albumin, etc.), amino acids (*e.g.,* aspartic acid, glutamic acid, lysine, arginine, glycine, histidine, etc.), fatty acids and phospholipids (*e.g*., alkyl sulfonates, caprylate, etc.), surfactants (*e.g*., SDS, polysorbate, nonionic surfactant, etc.), saccharides (*e.g.,* sucrose, maltose, trehalose, etc.) and polyols (*e.g.,* mannitol, sorbitol, etc.). See, also, Remington's Pharmaceutical Sciences (1990) Mack Publishing Co., Easton, Pa.

The phrases "respond to treatment with a HDAC inhibitor" or "respond to treatment with a HDAC6 inhibitor" or similar phrases refer to the clinical benefit imparted to a patient suffering from a disease or condition, such as cancer, from or as a result of the treatment with the HDAC inhibitor (*e.g*., a HDAC6 inhibitor). A clinical benefit includes a complete remission, a partial remission, a stable disease (without progression), progression-free survival, disease free survival, improvement in the time-to-progression (of the disease), improvement in the time-to-death, or improvement in the overall survival time of the patient from or as a result of the treatment with the HDAC inhibitor. There are criteria for determining a response to therapy and those criteria allow comparisons of the efficacy to alternative treatments (Slapak and Kufe, Principles of Cancer Therapy, in Harrisons's Principles of Internal Medicine, 13th edition, eds. Isselbacher et al., McGraw-Hill, Inc., 1994). For example, a complete response or complete remission of cancer is the disappearance of all detectable malignant disease. A partial response or partial remission of cancer may be, for example, an approximately 50 percent decrease in the product of the greatest perpendicular diameters of one or more lesions or where there is not an increase in the size of any lesion or the appearance of new lesions.

The term "progression of cancer" includes and may refer to metastasis, a recurrence of cancer, or an at least approximately 25 percent increase in the product of the greatest perpendicular diameter of one lesion or the appearance of new lesions. The progression of cancer is "inhibited" if recurrence or metastasis of the cancer is reduced, slowed, delayed, or prevented.

The term "biomarker RNA" is a RNA that is a useful indicator of treatment efficiency in multiple myeloma.

A "kit" is any manufacture (*e.g*., a package or container) comprising at least one reagent, *e.g.,* a detection agent, for specifically detecting a biomarker RNA.

The term "mRNA" refers to messenger RNA, which is a polynucleotide that encodes a polypeptide. Traditionally, the basic components of an mRNA molecule include at least a coding region, a 5'UTR, a 3'UTR, a 5'cap, and a poly-A tail. Messenger RNA is a large family of RNA molecules that convey genetic information from DNA to the ribosome, where they specify the amino acid sequence of the protein products of gene expression. Following transcription of primary transcript mRNA (pre-mRNA) by RNA polymerase, processed, mature mRNA is translated into a protein.

The term "miRNA" refers to microRNA, which is a small non-coding RNA molecule (approximately 22 nucleotides) found in plants and animals, which functions in transcriptional and post-transcriptional regulation of gene expression. Encoded by eukaryotic nuclear DNA, miRNAs function via base-pairing with complementary sequences within mRNA molecules, usually resulting in gene silencing via translational repression or target degradation. The human genome may encode over 1000 miRNAs, which may target about 60% of mammalian genes and are abundant in many human cell types.

The term "non-coding RNA" refers to a functional RNA molecule that is not translated into a protein. Less-frequently used synonyms are non-protein-coding RNA (npcRNA), non-messenger RNA (nmRNA), and functional RNA (fRNA). The term small RNA (sRNA) is often used for short ncRNAs. The DNA sequence from which a non-coding RNA is transcribed is often called an RNA gene. Non-coding RNA genes include highly abundant and functionally important RNAs, such as transfer RNA (tRNA) and ribosomal RNA (rRNA), as well as RNAs such as snoRNAs, microRNAs, siRNAs, snRNAs, exRNAs, and piRNAs and the long ncRNAs that include examples such as Xist and HOTAIR. The number of ncRNAs encoded within the human genome is unknown, however recent transcriptomic and bioinformatic studies suggest the existence of thousands of ncRNAs. Since many of the newly identified ncRNAs have not been validated for their function, it is possible that many are non-functional.

The term "nucleic acid" refers to deoxyribonucleotides, ribonucleotides, or modified nucleotides, and polymers thereof in single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, and peptide-nucleic acids (PNAs).

The term "nucleotide" is used as recognized in the art to include natural bases (standard) and modified bases. Such bases are generally located at the 1' position of a nucleotide sugar moiety. Nucleotides generally comprise a base, sugar, and a phosphate group. The nucleotides can be unmodified or modified at the sugar, phosphate, and/or base moiety (also referred to interchangeably as nucleotide analogs, modified nucleotides, nonnatural nucleotides, non-standard nucleotides and other; see, e.g., Eckstein, et al., International PCT Publication No. WO 92/07065; and Usman et al, International PCT Publication No. WO 93115187. There are several examples of modified nucleic acid bases known in the art, as summarized by Limbach, et al, Nucleic Acids Res. 22:2183, 1994. Some of the non-limiting examples of base modifications that can be introduced into nucleic acid molecules include, hypoxanthine, purine, pyridin-4-one, pyridin-2-one, phenyl, pseudouracil, 2,4,6-trimethoxy benzene, 3-methyl uracil, dihydrouridine, naphthyl, aminophenyl, 5-alkylcytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), propyne, and others (Burgin, et al., Biochemistry 35:14090, 1996).

The term "modified bases" means nucleotide bases other than adenine, guanine, cytosine, thymine, and uracil at the 1' position or their equivalents.

The term "modified nucleotide" refers to a nucleotide that has one or more modifications to the nucleoside, the nucleobase, pentose ring, or phosphate group. For example, modified nucleotides exclude ribonucleotides containing adenosine monophosphate, guanosine monophosphate, uridine monophosphate, and cytidine monophosphate, and deoxyribonucleotides containing deoxyadenosine monophosphate, deoxyguanosine monophosphate, deoxythymidine monophosphate, and deoxycytidine monophosphate. Modifications include those naturally occurring that result from modification by enzymes that modify nucleotides, such as methyltransferases. Modified nucleotides also include synthetic or non-naturally occurring nucleotides. Synthetic or non-naturally occurring modifications in nucleotides include those with 2' modifications, e.g., 2'-methoxyethoxy, 2'-fluoro, 2'-allyl, 2'-O-[2-(methylamino)-2-oxoethyl], 4'-thio, 4'-CH₂-O-2'-bridge, 4'-(CH₂)₂-O-2'-bridge, 2'-LNA, and 2'-O-(N-methylcarbamate) or those comprising base analogs. In connection with 2'-modified nucleotides described herein, the term "amino" means 2'-NH₂ or 2'-O-NH₂, which can be modified or unmodified. Such modified groups are described, e.g., in Eckstein et al., U.S.Pat. No. 5,672,695 and Matulic-Adamic et al., U.S. Pat. No. 6,248,878.

The term "base analog" refers to a heterocyclic moiety that is located at the 1' position of a nucleotide sugar moiety in a modified nucleotide that can be incorporated into a nucleic acid duplex (or the equivalent position in a nucleotide sugar moiety substitution that can be incorporated into a nucleic acid duplex). A base analog is generally either a purine or pyrimidine base, excluding the common bases guanine (G), cytosine (C), adenine (A), thymine (T), and uracil (U). Base analogs can duplex with other bases or base analogs in dsNAs. Base analogs include those useful in compounds and methods outside of the scope of the invention, e.g., those disclosed in U.S. Pat. Nos. 5,432,272 and 6,001,983 to Benner, and U.S. Patent Publication No. 2008/0213891 to Manoharan. Non-limiting examples of bases include hypoxanthine (I), xanthine (X), 3-β-D-ribofuranosyl-(2,6-diaminopyrimidine) (K), 3-β-D-ribofuranosyl-(1-methyl-pyrazolo[4,3-d]pyrimidine-5,7(4H,6H)-dione) (P), iso-cytosine (iso-C), iso-guanine (iso-G), 1-β-D-ribofuranosyl-(5-nitroindole), 1-β-D-ribofuranosyl-(3-nitropyrrole), 5-bromouracil, 2-aminopurine, 4-thio-dT, 7-(2-thienyl)-imidazo[4,5-b ]pyridine (Ds) and pyrrole-2-carbaldehyde (Pa), 2-amino-6-(2-thienyl)purine (S), 2-oxopyridine (Y), difluorotolyl, 4-fluoro-6-methylbenzimidazole, 4-methylbenzimidazole, 3-methyl isocarbostyrilyl, 5-methyl isocarbostyrilyl, and 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylindolyl, 4,6-dimethylindolyl, phenyl, napthalenyl, anthracenyl, phenanthracenyl, pyrenyl, stilbenzyl, tetracenyl, pentacenyl, and structural derivates thereof (Schweitzer et al., J. Org. Chem., 59:7238-7242 (1994); Berger et al., Nucleic Acids Research, 28(15):2911-2914 (2000); Moran et al., J. Am. Chem. Soc., 119:2056-2057 (1997); Morales et al., J. Am. Chem. Soc., 121:2323-2324 (1999); Guckian et al., J. Am. Chem. Soc., 118:8182-8183 (1996); Morales et al., J. Am. Chem. Soc., 122(6):1001-1007 (2000); McMinn et al., J. Am. Chem. Soc., 121:11585-11586 (1999); Guckian et al., J.Org. Chem., 63:9652-9656 (1998); Moran et al., Proc. Natl. Acad. Sci., 94: 10506-10511 (1997); Das et al., J. Chem. Soc., Perkin Trans., 1:197-206 (2002); Shibata et al., J. Chem. Soc., Perkin Trans., 1: 1605-1611 (2001); Wu et al., J. Am. Chem. Soc., 122(32):7621-7632 (2000); O'Neill et al., J. Org. Chem., 67:5869-5875 (2002); Chaudhuri et al., J. Am. Chem. Soc., 117:10434-10442 (1995); and U.S. Pat. No. 6,218,108). Base analogs may also be a universal base.

The term "universal base" refers to a heterocyclic moiety located at the 1' position of a nucleotide sugar moiety in a modified nucleotide, or the equivalent position in a nucleotide sugar moiety substitution, that, when present in a nucleic acid duplex, can be positioned opposite more than one type of base without altering the double helical structure (e.g., the structure of the phosphate backbone). Additionally, the universal base does not destroy the ability of the single stranded nucleic acid in which it resides to duplex to a target nucleic acid. The ability of a single stranded nucleic acid containing a universal base to duplex a target nucleic can be assayed by methods apparent to one in the art (*e.g.,* UV absorbance, circular dichroism, gel shift, single stranded nuclease sensitivity, etc.). Additionally, conditions under which duplex formation is observed may be varied to determine duplex stability or formation, *e.g*., temperature, as melting temperature (Tₘ) correlates with the stability of nucleic acid duplexes. Compared to a reference single stranded nucleic acid that is exactly complementary to a target nucleic acid, the single stranded nucleic acid containing a universal base forms a duplex with the target nucleic acid that has a lower Tₘ than a duplex formed with the complementary nucleic acid. However, compared to a reference single stranded nucleic acid in which the universal base has been replaced with a base to generate a single mismatch, the single stranded nucleic acid containing the universal base forms a duplex with the target nucleic acid that has a higher Tₘ than a duplex formed with the nucleic acid having the mismatched base.

Some universal bases are capable of base pairing by forming hydrogen bonds between the universal base and all of the bases guanine (G), cytosine (C), adenine (A), thymine (T), and uracil (U) under base pair forming conditions. A universal base is not a base that forms a base pair with only one single complementary base. In a duplex, a universal base may form no hydrogen bonds, one hydrogen bond, or more than one hydrogen bond with each of G, C, A, T, and U opposite to it on the opposite strand of a duplex. Optionally, the universal base does not interact with the base opposite to it on the opposite strand of a duplex. In a duplex, base pairing between a universal base occurs without altering the double helical structure of the phosphate backbone. A universal base may also interact with bases in adjacent nucleotides on the same nucleic acid strand by stacking interactions. Such stacking interactions stabilize the duplex, especially in situations where the universal base does not form any hydrogen bonds with the base positioned opposite to it on the opposite strand of the duplex. Non-limiting examples of universal-binding nucleotides include inosine, 1-β-D-ribo furanosyl-5-nitroindole, and/or 1-β-D-ribofuranosyl-3-nitropyrrole (U.S. Patent Publication No. 20070254362; Van Aerschot et al., An acyclic 5-nitroindazole nucleoside analogue as ambiguous nucleoside. Nucleic Acids Res. 1995; 23(21):4363-70; Loakes et al., 3-Nitropyrrole and 5-nitroindole as universal bases in primers for DNA sequencing and PCR. Nucleic Acids Res. 1995; 23(13):2361-6; Loakes and Brown, 5-Nitroindole as a universal base analogue. Nucleic Acids Res. 1994; 22(20):4039-43).

The term "oligonucleotide strand" means a single stranded nucleic acid molecule. An oligonucleotide may comprise ribonucleotides, deoxyribonucleotides, modified nucleotides (e.g., nucleotides with 2' modifications, synthetic base analogs, etc.) or combinations thereof. Such modified oligonucleotides can be preferred over native forms because of properties such as, for example, enhanced cellular uptake and increased stability in the presence of nucleases.

The term "ribonucleotide" encompasses natural and synthetic, unmodified and modified ribonucleotides. Modifications include changes to the sugar moiety, to the base moiety, and/or to the linkages between ribonucleotides in the oligonucleotide. As used herein, the term "ribonucleotide" specifically excludes a deoxyribonucleotide, which is a nucleotide possessing a single proton group at the 2' ribose ring position.

The term "deoxyribonucleotide" encompasses natural and synthetic, unmodified and modified deoxyribonucleotides. Modifications include changes to the sugar moiety, to the base moiety, and/or to the linkages between deoxyribonucleotide in the oligonucleotide.

The term "small molecule" refers to a non- peptidic, non-oligomeric organic compound either synthesized in the laboratory or found in nature. Small molecules, as used herein, can refer to compounds that are "natural product-like", however, the term "small molecule" is not limited to "natural product-like" compounds. Rather, a small molecule is typically characterized in that it contains several carbon-carbon bonds, and has a molecular weight of less than 1500. Examples of "small molecules" that occur in nature include, but are not limited to, taxol, dynemicin, and rapamycin.

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. Particularly, in embodiments the compound is at least 85% pure, more preferably at least 90% pure, more preferably at least 95% pure, and most preferably at least 99% pure.

The term "RNA" refers to ribonucleic acid. RNA is comprised of nucleic acids. RNA is assembled as a chain of nucleotides, and is usually single-stranded.

The term "specifically binds" refers to binding to a certain RNA and not to other RNAs.

The term "therapy" refers to any protocol, method, and/or agent that can be used in the prevention, management, treatment, and/or amelioration of a disease.

The term "therapeutically effective amount" means an amount of a drug that causes a measurable effect in a subject, such as an amount effective for killing or inhibiting the growth of tumor cells.

"Treat", "treating", and "treatment" refer to a method of alleviating or abating a disease and/or its attendant symptoms.

The term "treatment efficiency" means how well a drug is doing its job, *i.e,* acting upon a target to produce a therapeutic effect.

### miRNA/mRNA Array

The Human miRNome Complete RT² miRNA PCR Array profiles the expression of the 752 most abundantly expressed and best characterized microRNA (miRNA) sequences in the Human miRNA genome (miRNome), as annotated by the Sanger miRBase Release 14.

The Illumina Gene Expression Beadchip content provides genome-wide transcriptional coverage of well-characterized genes, gene candidates, and splice variants. Each array on the BeadChip targets more than 47,000 probes derived from the National Center for Biotechnology Information Reference Sequence (NCBI) RefSeq Release 38 (November 7, 2009) and other sources.

### Histone deacetylase (HDAC) inhibitors

An HDAC inhibitor useful in an miRNA/mRNA array method outside of the scope of the invention and in the Examples herein can be any HDAC inhibitor, such as a small molecule organic compound, an antibody, a siRNA, an aptamer, a nucleic acid, a protein, or a peptide.

The HDAC inhibitor used in the invention is an HDAC6 inhibitor. This means that the HDAC inhibitor selectively inhibits HDAC6 over other forms of HDAC.

Outside of the scope of the invention is the HDAC6 specific inhibitor compound of Formula I: 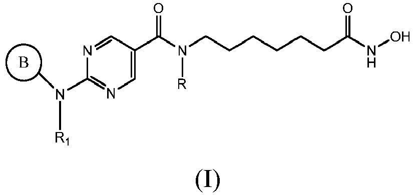
or a pharmaceutically acceptable salt thereof,
wherein,
ring B is aryl or heteroaryl;
Ri is an aryl or heteroaryl, each of which may be optionally substituted by OH, halo, or C₁₋₆₋alkyl;
   and
R is H or C₁₋₆₋alkyl.

The HDAC6 inhibitor of the invention is Compound A or Compound B:

| | |
|---|---|
| 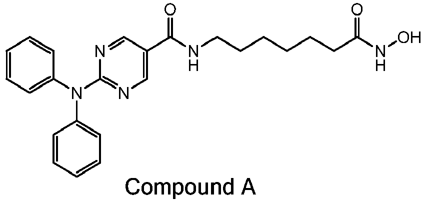 | 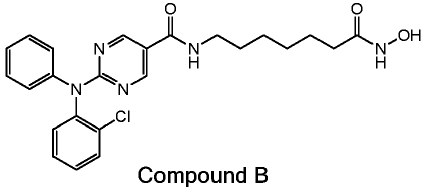 |
| 2-(diphenylamino)-N-(7-(hydroxyamino)-7-oxoheptyl)pyrimidine-5-carboxamide | 2-((2-chlorophenyl)(phenyl)amino)-N-(7-(hydroxyamino)-7-oxoheptyl)pyrimidine-5-carboxamide |
| IC₅₀(nM) HDAC6 = 10 HDAC3 = 84 | |
| | IC₅₀(nM) HDAC6 = 4 HDAC3 = 76 |

The preparation and properties of selective HDAC6 inhibitors according to Formula I are provided in International Patent Application No. PCT/US2011/021982.

Outside of the scope of the invention is the HDAC6 specific inhibitor compound of Formula II: 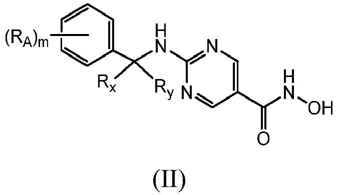
or a pharmaceutically acceptable salt thereof,
wherein,
Rₓ and R_{y} together with the carbon to which each is attached, form a cyclopropyl, cyclobutyl, cyclopentyl, cyclohexyl, cycloheptyl, or cyclooctyl;
each R_{A} is independently C₁₋₆₋alkyl, C₁₋₆₋alkoxy, halo, OH, -NO₂, -CN, or - NH₂; and
m is 0, 1, or 2.

Representative compounds of Formula II include, but are not limited to:

**or pharmaceutically acceptable salts thereof.**

| | |
|---|---|
| 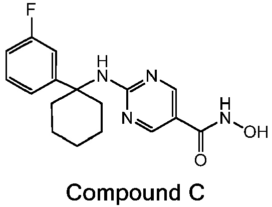 | 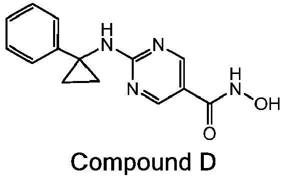 |
| IC₅₀(nM) HDAC6 = 7 HDAC1 = 2123 | IC₅₀(nM) HDAC6 = 2 HDAC1 = 94 (60x) |
| (283.5x) HDAC2 = 2570 (9343.2x) | HDAC2 = 128 (81.9x) HDAC3=219 |
| HDAC3=11223 (1498.8x) | (139.5x) |

The preparation and properties of selective HDAC6 inhibitors according to Formula II are provided in International Patent Application No. PCT/US2011/060791.

HDAC inhibitors have one or more of the following properties: the compound is capable of inhibiting at least one histone deacetylase; the compound is capable of inhibiting HDAC6; the compound is a selective HDAC6 inhibitor; the compound binds to the polyubiquitin binding domain of HDAC6; the compound is capable of inducing apoptosis in cancer cells (especially multiple myeloma cells, non-Hodgkin's lymphoma (NML) cells, breast cancer cells, acute myelogeous leukemia (AML) cells); and/or the compound is capable of inhibiting aggresome formation.

An HDAC inhibitor may comprise a metal binding moiety, such as a zinc-binding moiety such as a hydroxamate. Certain hydroxamates are potent inhibitors of HDAC6 activity; without wishing to be bound by theory, it is believed that the potency of these hydroxamates is due, at least in part, to the ability of the compounds to bind zinc. An HDAC inhibitor may include at least one portion or region that can confer selectivity for a biological target implicated in the aggresome pathway, e.g., a biological target having tubulin deacetylase (TDAC) or HDAC activity, e.g., HDAC6. Thus, some HDAC inhibitors include a zinc-binding moiety spaced from other portions of the molecule that are responsible for binding to the biological target.

### Biomarkers

As shown in the Examples herein, the miRNA/mRNA array methods outside of the scope of the invention may be used to identify biomarkers that tell us why a particular set of cells is killed. That is, the biomarkers are indicative of HDAC inhibitors and cell death in myeloma.

The set of cells may contain a control group of cells and a test group of cells. This set of cells allows one to determine how a particular drug works in a particular type of cancer cell.

Outside of the scope of the invention is a histone deacetylase 6 (HDAC6) biomarker RNA for multiple myeloma. The biomarker RNA comprises a nucleic acid sequence selected from the group consisting of SEQ ID NOs: 1-25 or 27.

Outside of the scope of the invention, the biomarker RNA is a miRNA comprising a nucleic acid sequence selected from the group consisting of SEQ ID NOs: 1-23.

Outside of the scope of the invention, the miRNA comprising a nucleic acid sequence selected from the group consisting of SEQ ID NOs: 1-11 can be down-regulated by a HDAC6 inhibitor (e.g., Compound D). Those miRNAs can be down-regulated by 3-fold or more by a HDAC6 inhibitor.

Outside of the scope of the invention, the miRNA comprising a nucleic acid sequence selected from the group consisting of SEQ ID NOs: 12-23 can be up-regulated by a HDAC6 inhibitor (*e.g*., Compound D). Those miRNAs can be up-regulated by 3-fold or more by a HDAC6 inhibitor.

Outside of the scope of the invention, the biomarker RNA is a mRNA comprising a nucleic acid sequence selected from the group consisting of SEQ ID NOs: 24-25.

The mRNA comprising a nucleic acid sequence of SEQ ID NO: 24 can be down-regulated by a HDAC6 inhibitor (*e.g*., Compound D). The mRNA can be down-regulated by 2-fold or more by a HDAC6 inhibitor.

The mRNA comprising a nucleic acid sequence of SEQ ID NO: 25 can be up-regulated by a HDAC6 inhibitor (*e.g*., Compound D). The mRNA can be up-regulated by 2-fold or more by a HDAC6 inhibitor.

Outside of the scope of the invention, the biomarker RNA is a small non-coding RNA comprising a nucleic acid sequence selected from the group consisting of SEQ ID NO: 27. In the invention, the HDAC6 biomarker RNA consists of the nucleic acid sequence of SEQ ID NO: 26.

The small non-coding RNA comprising a nucleic acid sequence selected from the group consisting of SEQ ID NOs: 26-27 can be down-regulated by a HDAC6 inhibitor (*e.g*., Compound D). In a specific embodiment, the small non-coding RNA is down-regulated by 2 fold or more by the HDAC6 inhibitor.

### Kits

Outside of the scope of the invention are kits that may be used in the experimental methods in order to identify a histone deacetylase 6 (HDAC6) inhibitor, or to identify drug specific and/or disease specific biomarkers.

Outside of the scope of the invention is a kit for determining the treatment efficiency of a histone deacetylase 6 (HDAC6) inhibitor in a subject having multiple myeloma comprising: a detection agent that specifically binds to a HDAC6 biomarker RNA (ribonucleic acid) selected from the group consisting of SEQ ID NOs: 1-27; and instructions for measuring the expression level of a HDAC6 biomarker RNA comprising a nucleic acid sequence selected from the group consisting of SEQ ID NOs: 1-27. The HDAC6 biomarker RNA can be one of the HDAC6 biomarker RNAs as provided herein. The cell can be a myeloma cell (*e.g.,* MM.1S or RPMI8226) or a bone marrow stromal cell (*e.g.,* HS-5).

Outside of the scope of the invention is a kit for determining the treatment efficiency of a histone deacetylase 6 (HDAC6) inhibitor in a subject having multiple myeloma comprising: one or more detection agents that specifically bind to a HDAC6 biomarker RNA (ribonucleic acid) consisting of at least 2 of, at least 3 of, at least 4 of, at least 5 of, at least 6 of, at least 7 of, at least 8 of, at least 9 of, at least 10 of, at least 11 of, at least 12 of, at least 13 of, at least 14 of, at least 15 of, at least 16 of, at least 17 of, at least 18 of, at least 19 of, at least 20 of, at least 21 of, at least 22 of, at least 23 of, at least 24 of, at least 25 of, at least 26 of, or at least 27 of the nucleic acids of SEQ ID NOs: 1-27; and instructions for measuring the expression level of a HDAC6 biomarker RNA comprising a nucleic acid sequence selected from the group consisting of SEQ ID NOs: 1-27. The cell can be a myeloma cell (*e.g.,* MM.1S or RPMI8226) or a bone marrow stromal cell (*e.g.,* HS-5).

Outside of the scope of the invention is a kit for identifying a histone deacetylase 6 (HDAC6) inhibitor that is useful in the treatment of multiple myeloma comprising: a multiple myeloma cell or a bone marrow stromal cell; a detection agent that specifically binds to a HDAC6 biomarker RNA (ribonucleic acid) selected from the group consisting of SEQ ID NOs: 1-27; and instructions for measuring the expression level of a HDAC6 biomarker RNA comprising a nucleic acid sequence selected from the group consisting of SEQ ID NOs: 1-27. The myeloma cell can be a MM.1S cell or a RPMI8226 cell. The bone marrow stromal cell can be a HS-5 cell.

### Methods

The invention provides a method for monitoring the treatment efficiency of a HDAC6 inhibitor in a subject. The subject can be a cell or a mammal.

Outside of the scope of the invention is a method for monitoring the treatment efficiency of a histone deacetylase 6 (HDAC6) inhibitor in a subject comprising:
a) administering a therapeutically effective amount of an HDAC inhibitor to a subject;
b) taking a biological sample from the subject;
c) determining the amount of an HDAC6 biomarker RNA comprising a nucleic acid sequence selected from the group consisting of SEQ ID NOs: 1-25 and 27 in the sample; and
d) concluding that the HDAC6 treatment is efficient if an HDAC6 biomarker RNA comprising a nucleic acid sequence selected from the group consisting of SEQ ID NOs: 1-11, 24, and 27 is down-regulated, and/or if an HDAC6 biomarker RNA comprising a nucleic acid sequence selected from the group consisting of SEQ ID NOs: 12-23 and 25 is up-regulated.

The method will monitor whether the HDAC treatment that the subject receives is efficient. That is, the method will be indicative of HDAC6 inhibition and cell death.

Outside of the scope of the invention, the HDAC6 inhibitor may be any HDAC6 inhibitor known in the art, including Compound A or Compound D.

The HDAC6 inhibitor may be administered using any therapeutically effective amount and any route of administration.

The sample can be a myeloma cell (*e.g.,* MM.1S or RPMI8226) or a bone marrow stromal cell (*e.g.,* HS-5).

The subject can also be a mammal.

The method involves taking a biological sample from a subject (*e.g.,* a human) in order to determine the treatment efficiency of the HDAC6 inhibitor in the subject with a particular disease. For example, the biological sample may be a sample from whole blood, blood serum, blood plasma, semen, urine, mucus, bone marrow, or other body sample. In one embodiment, the biological sample is a bone marrow sample. In another embodiment, the biological sample is a myeloma sample.

Outside of the scope of the invention, step d) comprises concluding that the HDAC6 treatment is efficient if a HDAC6 biomarker RNA comprising a nucleic acid sequence selected from the group consisting of SEQ ID NOs: 1-11 is down-regulated by 3 fold or more.

Ouside of the scope of the invention, step d) comprises concluding that the HDAC6 treatment is efficient if a HDAC6 biomarker RNA comprising a nucleic acid sequence selected from the group consisting of SEQ ID NOs: 24 and 27 is down-regulated by 2 fold or more.

Outside of the scope of the invention, step d) comprises concluding that the HDAC6 treatment is efficient if a HDAC6 biomarker RNA comprising a nucleic acid sequence selected from the group consisting of SEQ ID NOs: 12-23 is up-regulated by 3 fold or more.

Outside of the scope of the invention, step d) comprises concluding that the HDAC6 treatment is efficient if a HDAC6 biomarker RNA comprising a nucleic acid sequence selected from the group consisting of SEQ ID NO: 25 is up-regulated by 2 fold or more.

The determining step may use any means or detection agent known in the art to identify the histone deacetylase 6 (HDAC6) biomarker RNA used in the invention.

In yet another embodiment of the invention, the method may further comprise step d) treating the subject with additional HDAC6 inhibitor if it determined in step c) that the HDAC6 treatment is not efficient.

Outside of the scope of the invention is a method for treating a subject with multiple myeloma who expresses one or more of the histone deacetylase 6 (HDAC6) biomarker ribonucleic acids (RNAs) selected from the group consisting of SEQ ID NOs: 1-27 comprising admininstering to the subject an HDAC6 inhibitor. The HDAC6 biomarker RNA of SEQ ID NOs: 1-11, 24, or 26-27 may be down-regulated, and/or the HDAC6 biomarker RNA of SEQ ID NOs: 12-23 or 25 may be up-regulated.

### EXAMPLES

Examples denoted ^{∗} are outside of the scope of the invention. Examples have been set forth below for the purpose of illustration and to describe certain specific embodiments of the invention. However, the scope of the claims is not to be in any way limited by the examples set forth herein. Various changes and modifications to the disclosed embodiments will be apparent to those skilled in the art and such changes and modifications may be made without departing from the scope of the appended claims. Definitions of the variables in the structures in the schemes herein are commensurate with those of corresponding positions in the formulae presented herein.

Conventional techniques of molecular biology and nucleic acid/protein chemistry, which are within the skill of the art, are explained in the literature and used in the practice of the invention. See, for example, Sambrook, J. et al., Molecular Cloning: A Laboratory Manual, Cold Spring Harbor Laboratory Press, Cold Spring Harbor, N.Y., 1989; Gait, M. J. (ed.), Oligonucleotide synthesis--a practical approach, IRL Press Limited, 1984; Hames, B. D. and Higgins, S. J. (eds.), Nucleic acid hybridisation--a practical approach, IRL Press Limited, 1985; and a series, Methods in Enzymology, Academic Press, Inc.

The synthesis of the compounds of Formula I is provided in PCT/US2011/021982. The synthesis of compounds of Formula II is provided in PCT/US2011/060791. The synthesis of the compounds of Formula III is provided in U.S. Patent Nos. 5,635,517; 6,281,230; 6,335,349; and 6,476,052; and in International Patent Application No. PCT/US97/013375.

### Example 1: Synthesis of 2-(diphenylamino)-N-(7-(hydroxyamino)-7-oxoheptyl) pyrimidine-5-carboxamide (Compound A)

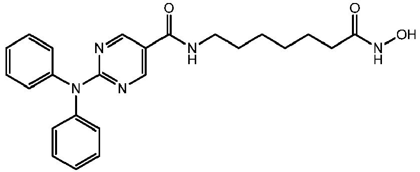

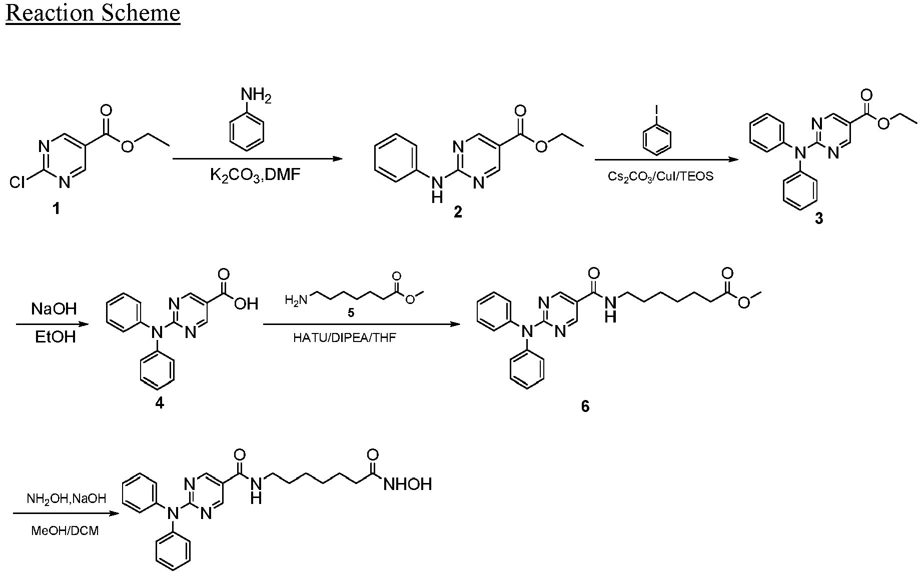

### Synthesis of Intermediate 2

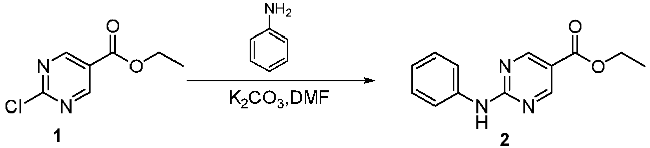

A mixture of aniline (3.7 g, 40 mmol), ethyl 2-chloropyrimidine-5-carboxylate **1** (7.5 g, 40 mmol), K₂CO₃ (11 g, 80 mmol) in DMF (100 ml) was degassed and stirred at 120 °C under N₂ overnight. The reaction mixture was cooled to rt and diluted with EtOAc (200 ml), then washed with saturated brine (200 ml × 3). The organic layer was separated and dried over Na₂SO₄, evaporated to dryness and purified by silica gel chromatography (petroleum ethers/EtOAc = 10/1) to give the desired product as a white solid (6.2 g, 64 %).

### Synthesis of Intermediate 3

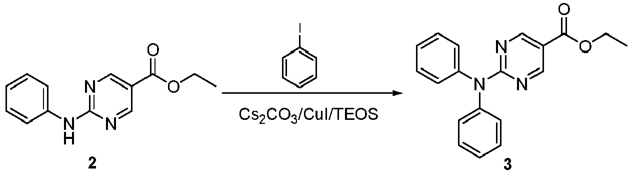

A mixture of the compound **2** (6.2 g, 25 mmol), iodobenzene (6.12 g, 30 mmol), CuI (955 mg, 5.0 mmol), Cs₂CO₃ (16.3 g, 50 mmol) in TEOS (200 ml) was degassed and purged with nitrogen. The resulting mixture was stirred at 140 °C for 14h. After cooling to rt, the residue was diluted with EtOAc (200 ml) and 95%EtOH (200 ml), NH₄F-H₂O on silica gel [50g, pre-prepared by the addition of NH₄F (100g) in water (1500 ml) to silica gel (500g, 100-200mesh)] was added, and the resulting mixture was kept at rt for 2 h, the solidified materials was filtered and washed with EtOAc. The filtrate was evaporated to dryness and the residue was purified by silica gel chromatography (petroleum ethers/EtOAc = 10/1) to give a yellow solid (3 g, 38 %).

### Synthesis of Intermediate 4

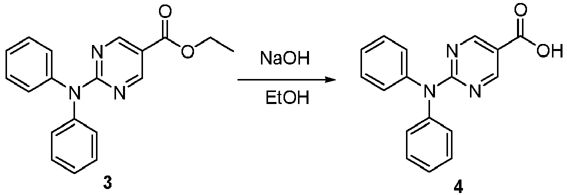

2N NaOH (200 ml) was added to a solution of the compound **3** (3.0 g, 9.4 mmol) in EtOH (200 ml). The mixture was stirred at 60°C for 30min. After evaporation of the solvent, the solution was neutralized with 2N HCl to give a white precipitate. The suspension was extracted with EtOAc (2 × 200 ml), and the organic layer was separated, washed with water (2 × 100 ml), brine (2 × 100 ml), and dried over Na₂SO₄. Removal of solvent gave a brown solid (2.5 g, 92 %).

### Synthesis of Intermediate 6

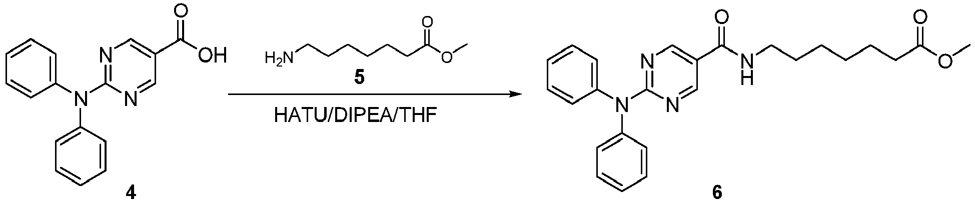

A mixture of compound **4** (2.5 g, 8.58 mmol), aminoheptanoate **5** (2.52 g, 12.87 mmol), HATU (3.91 g, 10.30 mmol), DIPEA (4.43 g, 34.32 mmol) was stirred at rt overnight. After the reaction mixture was filtered, the filtrate was evaporated to dryness and the residue was purified by silica gel chromatography (petroleum ethers/EtOAc = 2/1) to give a brown solid (2 g, 54 %).

### Synthesis of 2-(diphenylamino)-N-(7-(hydroxyamino)-7-oxoheptyl)pyrimidine-5-carboxamide

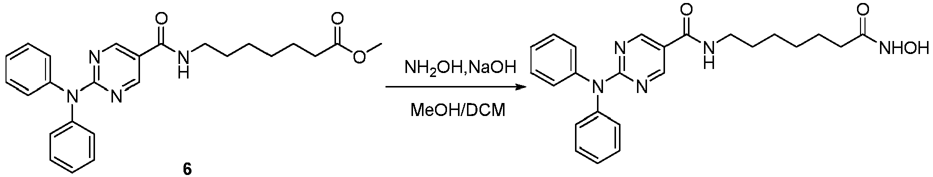

A mixture of the compound **6** (2.0 g, 4.6 mmol), sodium hydroxide (2N, 20 mL) in MeOH (50 ml) and DCM (25 ml) was stirred at 0 °C for 10min. Hydroxylamine (50%) (10 ml) was cooled to 0 °C and added to the mixture. The resulting mixture was stirred at rt for 20min. After removal of the solvent, the mixture was neutralized with 1M HCl to give a white precipitate. The crude product was filtered and purified by pre-HPLC to give a white solid (950 mg, 48%).

### Example 2: Synthesis of 2-((2-chlorophenyl)(phenyl)amino)-N-(7-(hydroxyamino)-7-oxoheptyl)pyrimidine-5-carboxamide (Compound B)

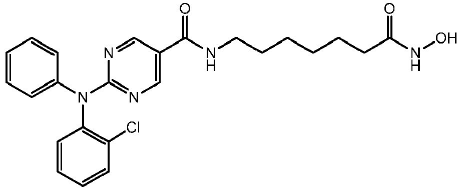

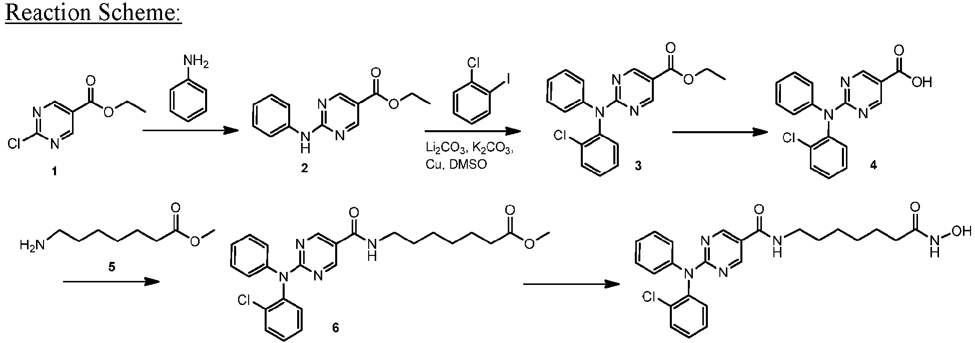

Synthesis of Intermediate 2: See synthesis of intermediate **2** in Example 1.

Synthesis of Intermediate 3: A mixture of compound **2** (69.2 g, 1 equiv.), 1-chloro-2-iodobenzene (135.7 g, 2 equiv.), Li₂CO₃ (42.04 g, 2 equiv.), K₂CO₃ (39.32 g, 1 equiv.), Cu (1 equiv. 45 µm) in DMSO (690 ml) was degassed and purged with nitrogen. The resulting mixture was stirred at 140 ° C. Work-up of the reaction gave compound **3** at 93 % yield.

Synthesis of Intermediate 4: See synthesis of intermediate 4 in Example 1.

Synthesis of Intermediate 6: See synthesis of intermediate 6 in Example 1.

Synthesis of 2-((2-chlorophenyl)(phenyl)amino)-N-(7-(hydroxyamino)-7-oxoheptyl)pyrimidine-5-carboxamide **(Compound B**): See synthesis of **Compound A** in Example 1.

### * Example 3: Synthesis of 2-((1-(3-fluorophenyl)cyclohexyl)amino)-N-hydroxypyrimidine-5-carboxamide (Compound C)

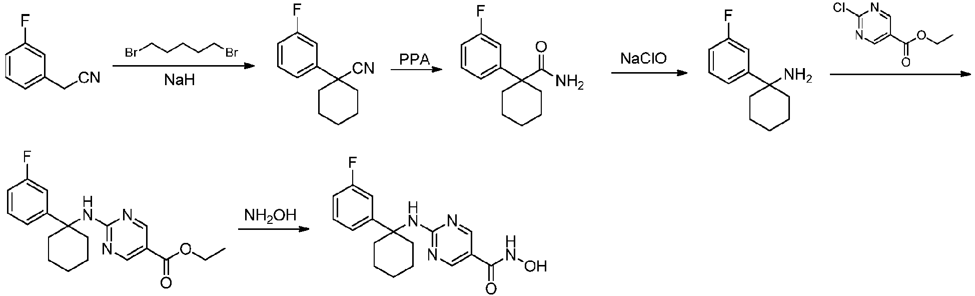

### Synthesis of 1-(3-fluorophenyl)cyclohexanecarbonitrile:

To a solution of 2-(3-fluorophenyl)acetonitrile (100 g, 0.74 mol) in Dry DMF (1000 ml) was added 1,5-dibromopentane (170 g, 0.74 mol), NaH (65 g, 2.2 eq) was added dropwise at ice bath. After addition, the resulting mixture was vigorously stirred overnight at 50°C. The suspension was quenched by ice water carefully, extracted with ethyl acetate (3^{∗}500 ml). The combined organic solution was concentrate to afford the crude which was purified on flash column to give 1-(3-fluorophenyl)cyclohexanecarbonitrile as pale solid (100 g, 67%).

### Synthesis of 1-(3-fluorophenyl)cyclohexanecarboxamide:

To a solution of 1-(3-fluorophenyl)cyclohexanecarbonitrile (100 g, 0.49 mol) in PPA (500 ml) was heated at 110°C for about 5-6 hours. After completed, the resulting mixture was carefully basified with sat.NaHCO3 soultion until the PH=8-9. The precipitate was collected and washed with water (1000 ml) to afford 1-(3-fluorophenyl)cyclohexanecarboxamide as white solid (95 g, 87%).

### Synthesis of 1-(3-fluorophenyl)cyclohexanamine:

To a solution of 1-(3-fluorophenyl)cyclohexanecarboxamide (95 g, 0.43 mol) in n-BuOH (800 ml) was added NaClO (260 ml, 1.4 eq), then 3N NaOH (400 ml, 2.8 eq) was added at 0⁰C and the reaction was stirred overnight at r.t. The resulting mixture was extracted with EA (2^{∗}500 ml), the combined organic solution was washed with brine, dried to afford the crude which was further purification on treating with HCl salt as white powder (72 g, 73%).

### Synthesis of ethyl 2-(1-(3-fluorophenyl)cyclohexylamino)pyrimidine-5-carboxylate:

To a solution of 1-(3-fluorophenyl)cyclohexanamine hydrochloride (2.29 g 10 mmol) in Dioxane (50 ml) was added ethyl 2-chloropyrimidine-5-carboxylate (1.87 g, 1.0 eq) and DIPEA (2.58 g, 2.0 eq). The mixture was heated overnight at 110-120⁰C. The resulting mixture was directly purified on silica gel column to afford the coupled product as white solid (1.37 g, 40%)

### Synthesis of 2-((1-(3-fluorophenyl)cyclohexyl)amino)-N-hydroxypyrimidine-5-carboxamide:

To a solution of ethyl 2-(1-(3-fluorophenyl)cyclohexylamino)pyrimidine-5-carboxylate (100 mg, 0.29 mmol) in MeOH/DCM(10 ml, 1:1) was added 50% NH₂OH in water (2 ml, excess), then sat. NaOH in MeOH (2 ml, excess) was added at 0⁰C and the reaction was stirred for 3-4 hours. After completed, the resulting mixture was concentrated and acidified with 2N HCl to the PH=4-5. The precipitate was collected and washed by water (10 ml) to remove the NH₂OH and dried to afford 2-((1-(3-fluorophenyl)cyclohexyl)amino)-N-hydroxypyrimidine-5-carboxamide as white powder (70 mg, 73%).

### * Example 4: Synthesis of N-hydroxy-2-((1-phenylcyclopropyl)amino)pyrimidine-5-carboxamide (Compound D)

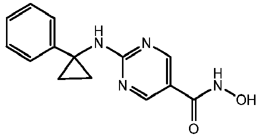

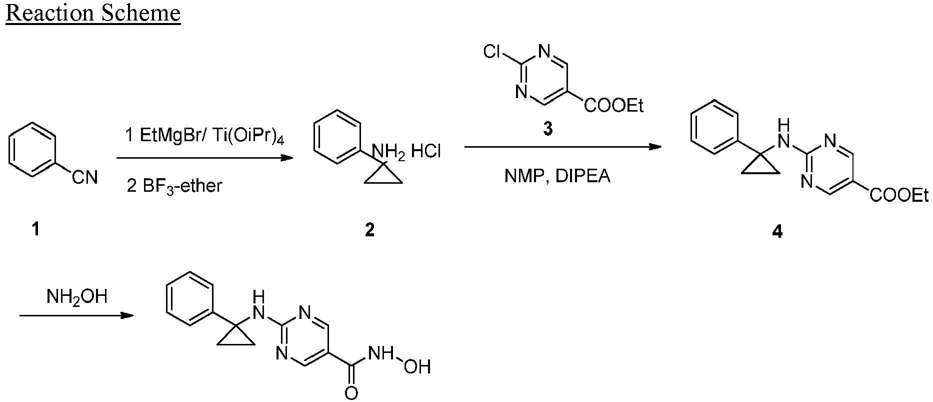

Synthesis of Intermediate 2: A solution of compound **1**, benzonitrile, (250 g, 1.0 equiv.), and Ti(OiPr)₄ (1330 ml, 1.5 equiv.) in MBTE (3750 ml) was cooled to about -10 to - 5 °C under a nitrogen atmosphere. EtMgBr (1610 ml, 3.0M, 2.3 equiv.) was added dropwise over a period of 60 min., during which the inner temperature of the reaction was kept below 5 °C. The reaction mixture was allowed to warm to 15-20 °C for 1 hr. BF₃-ether (1300 ml, 2.0 equiv.) was added dropwise over a period of 60 min., while the inner temperature was maintained below 15 °C. The reaction mixture was stirred at 15-20 °C for 1-2 hr. and stopped when a low level of benzonitrile remained. 1N HCl (2500 ml) was added dropwise while maintaining the inner temperature below 30 °C. NaOH (20%, 3000 ml) was added dropwise to bring the pH to about 9.0, while still maintaining a temperature below 30 °C. The reaction mixture was extracted with MTBE (3 L × 2) and EtOAc (3 L × 2), and the combined organic layers were dried with anhydrous Na₂SO₄ and concentrated under reduced pressure (below 45 °C) to yield a red oil. MTBE (2500 ml) was added to the oil to give a clear solution, and upon bubbling with dry HCl gas, a solid precipitated. This solid was filtered and dried in vacuum yielding 143 g of compound **2.**

Synthesis of Intermediate 4: Compound **2** (620 g, 1.0 equiv) and DIPEA (1080 g, 2.2 equiv. were dissolved in NMP (3100 ml) and stirred for 20 min. Compound **3** (680 g, 1.02 equiv.) was added and the reaction mixture was heated to about 85-95 °C for 4 hrs. The solution was allowed to slowly cool to r.t. This solution was poured onto H₂O (20 L) and much of the solid was precipitated out from the solution with strong stirring. The mixture was filtered and the cake was dried under reduced pressure at 50 °C for 24 hr., yielding 896 g of compound **4** (solid, 86.8%).

Synthesis of N-hydroxy-2-((1-phenylcyclopropyl)amino)pyrimidine-5-carboxamide **(Compound D):** A solution of MeOH(1000 ml) was cooled to about 0-5 °C with stirring. NH₂OH HCl (1107 g, 10 equiv.) was added, followed by careful addition of NaOCH₃ (1000 g, 12.0 equiv.) The resulting mixture was stirred at 0-5 °C for one hr, and was filtered to remove the solid. Compound **4** (450 g, 1.0 equiv.) was added to the reaction mixture in one portion, and stirred at 10 °C for two hours until compound **4** was consumed. The reaction mixture was adjusted to a pH of about 8.5-9 through addition of HCl (6N), resulting in precipitation. The mixture was concentrated under reduced pressure. Water (3000 ml) was added to the residue with intense stirring and the precipitate was collected by filtration. The product was dried in an oven at 45 °C overnight (340 g, 79% yield).

### Example 5 - microRNA and mRNA Array

Compound D, which is outside of the scope of the invention, was incubated with a multiple myeloma cell line (MM. 1S or RPMI8226) or a stromal cell line (HS-5) at 37°C for 6 hours. The concentration of Compound D was 2 uM.

Alternatively, Compound A was incubated with the multiple myeloma cell line MM.1S at at 37°C for 6 hours.

Total RNA was extracted from the treated cells (*e.g.,* MM.1S cells and RPMI8226 cells) and analyzed using microRNA array.

The results of these studies are shown in **Table 1** below.

The first column of **Table 1** shows the cells that were treated with Compound A or Compound D.

The second column of **Table 1** shows the RNA biomarkers identified, including miRNAs and mRNAs, in the corresponding cells of column 1.

The third column (Compound D) and fourth column (Compound A) of **Table 1** shows the expression of the RNA biomarkers in the treated cells as compared to control cells. The numbers shown are normalized to the expression level of the corresponding biomarker in control cells. For example, the expression level of biomarker hsa-miR-346 was 0.26, which means that the expression level of this biomarker in treated MM.1S cells was 26% of the expression level of this biomarker in MM. 1S control cells. Conversely, the expression level of biomarker hsa-miR-145 was 3.09, which means that the expression level of this biomarker in treated MM.1S cells was 3.09 fold of the expression level of this biomarker in MM.1S control cells. Thus, a number less than 1 indicates that the corresponding biomarker is down-regulated by Compound D, and a number greater than 1 indicates that the corresponding biomarker is up-regulated by Compound D.

In Stromal HS-5 cells, the biomarker HIF-1a was down-regulated by Compound D because its expression level in treated cells was 49% of the expression level in control cells. Conversely, biomarker PTPRU was up-regulated by Compound D because its expression level in treated cells was 2.52 fold of the expression level in control cells.

**Table 1. Expression of miRNAs and mRNAs in myeloma cells and stromal cells treated with Compound A**

| Cell line | miRNA / mRNA | * Cmpd D | Cmpd A |
|---|---|---|---|
| MM.1S | 5'- UGUCUGCCCGCAUGCCUGCCUCU -3' (SEQ ID NO: 1) | 0.26 | |
| | 5'- AGGAGGCAGCGCUCUCAGGAC -3' (SEQ ID NO: 2) | 0.33 | |
| | 5'- UGAAGGUCUACUGUGUGCCAGG -3' (SEQ ID NO: 3) | 0.23 | |
| | 5'- GGGGAGCTGTGGAAGCAGTAAA -3' (SEQ ID NO: 4) | 0.27 | |
| | 5'- CGTGCCACCCTTTTCCCCAG -3' (SEQ ID NO: 5) | 0.31 | |
| | 5'- GTCCAGTTTTCCCAGGAATCCCT -3' (SEQ ID NO: 12) | 3.09 | |
| | 5'- CGTAGAACCGACCTTGCG -3' (SEQ ID NO: 13) | 3.53 | |
| | 5'- TTGCAGCTGCCTGGGAGTGACTTC -3' (SEQ ID NO: 14) | 3.19 | |
| | 5'- AGGCATTGACTTCTCACTAGCT -3' (SEQ ID NO: 15) | 3.29 | |
| | 5'- CCTGTTGAAGTGTAATCCCCAAA -3' (SEQ ID NO: 16) | 4.68 | |
| | 5’- GTCGTCAAAGGTTACAAAGGCAAAGCCCCTTTTCT TGCCACTGCCTCGGT-3’ (SEQ ID NO: 26) | 0.46 | 0.74 |
| | 5’- AAGTTGATTTAACATTGTCTCCCCCCACAACCGCG CTTGACTAGCTTGCT -3’ (SEQ ID NO: 27) | 0.45 | 0.74 |
| RPMI-8226 | 5'- AUGACCUAUGAAUUGACAGAC-3' (SEQ ID NO: 6) | 0.28 | |
| | 5'- AGGAGGCAGCGCUCUCAGGAC -3' (SEQ ID NO: 2) | 0.09 | |
| | 5'- AAAGCGCUUCUCUUUAGAGGA -3' (SEQ ID NO: 7) | 0.25 | |
| | 5'- UACUCAAAAAGCUGUCAGUCA -3' (SEQ ID NO: 8) | 0.33 | |
| | 5'- CAGGAUGUGGUCAAGUGUUGUU -3' (SEQ ID NO: 9) | 0.24 | |
| | 5'- CUGGACUGAGCCAUGCUACUGG -3' (SEQ ID NO: 17) | 3.22 | |
| | 5'- GGGCGACAAAGCAAGACUCUUUCUU -3' (SEQ ID NO: 18) | 3.31 | |
| Stromal HS-5 | 5'-TCACTCCTCTCCTCCCGTCTT -3' (SEQ ID NO: 10) | 0.26 | |
| | 5'- CTTCCTCGTCTGTCTGCCCCAA -3' (SEQ ID NO: 11) | 0.15 | |
| | 5'- UAUGGCUUUUUAUUCCUAUGUGA -3' (SEQ ID NO: 19) | 6.64 | |
| | 5'-TAATCTCAGCTGGCAACTGTGAAA-3' (SEQ ID NO: 20) | 21.71 | |
| | 5'-ATCGGGAATGTCGTGTCCGCC-3' (SEQ ID NO: 21) | 54.35 | |
| | 5'- CTGTACTGAGCTGCCCCGAGAA-3' (SEQ ID NO: 22) | 5.95 | |
| | 5'- CGGAGAGGGCCCACAGTGAA -3' (SEQ ID NO: 23) | 3.52 | |
| | 5’- CCTAAATGTTCTGCCTACCCTGTTGGTATAAAGATA TTTTGAGCAGACTG-3’ (SEQ ID NO: 24) | 0.49 | |
| | 5’- TCAGGCTGCCCGTTGTGGGGAGGGGCAGTGTTAGA GCAGGGCTGGTCATA-3’ (SEQ ID NO: 25) | 2.52 | |

## Claims

1. A method for monitoring the treatment efficiency of a histone deacetylase 6 (HDAC6) inhibitor in a subject comprising:
a) providing a biological sample having been taken from a subject who has received a therapeutically effective amount of an HDAC6 inhibitor;
b) determining the amount of the HDAC6 biomarker RNA (ribonucleic acid) consisting of the nucleic acid sequence of SEQ ID NO: 26 in the sample; and
c) concluding that the HDAC6 treatment is efficient if the HDAC6 biomarker RNA consisting of the nucleic acid sequence of SEQ ID NO: 26 is down-regulated;
wherein the HDAC6 inhibitor is Compound A 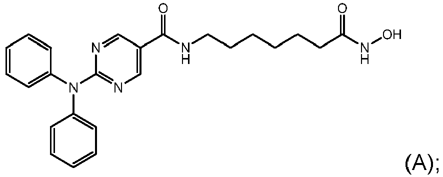 or Compound B

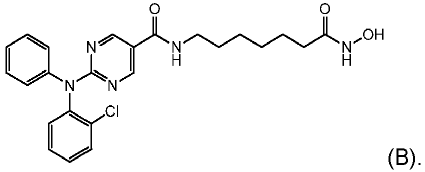

2. The method of claim 1, wherein the sample is a myeloma sample, or a bone marrow sample.

3. The method of claim 1, wherein step c) comprises concluding that the HDAC6 treatment is efficient if the HDAC6 biomarker RNA consisting of the nucleic acid sequence of SEQ ID NO: 26 is down-regulated by 2-fold or more

4. The method of any one of claims 1-3, wherein the HDAC6 inhibitor is Compound A

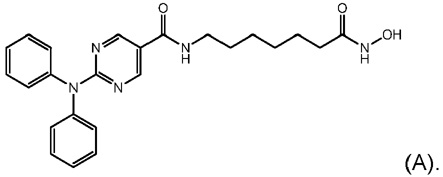

5. The method of any one of claims 1-3, wherein the HDAC6 inhibitor is Compound B

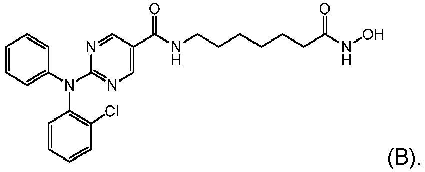

6. A HDAC6 inhibitor for use in the treatment of a subject, wherein the treatment efficiency of the HDAC6 inhibitor in the subject is monitored using the method of claim 1, wherein the treatment further comprises an additional amount of the HDAC6 inhibitor if the method of claim 1 determined in step c) that the HDAC6 treatment is not efficient,
wherein the HDAC6 inhibitor is Compound A 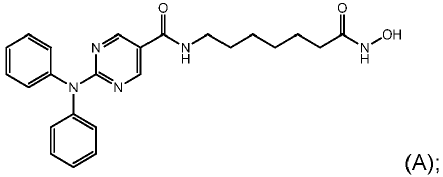 or Compound B

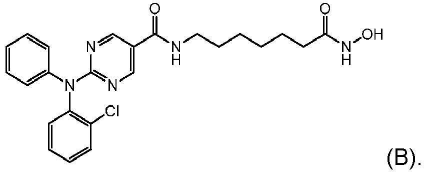

## Patentansprüche

1. Verfahren zum Überwachen der Behandlungseffizienz eines Histondeacetylase 6 (HDAC6)-Inhibitors bei einem Individuum, umfassend:
a) Bereitstellen einer biologischen Probe, die von einem Individuum entnommen wurde, das eine therapeutisch wirksame Menge eines HDAC6-Inhibitors erhalten hat;
b) Bestimmen der Menge der HDAC6-Biomarker-RNA (Ribonukleinsäure), bestehend aus der Nukleinsäuresequenz SEQ ID NR:26, in einer Probe; und
c) Feststellen, dass die HDAC6-Behandlung wirksam ist, wenn die HDAC6-Biomarker-RNA, bestehend aus der Nukleinsäuresequenz SED ID NR:26, herunterreguliert ist;
wobei es sich bei dem HDAC6-Inhibitor um
Verbindung A 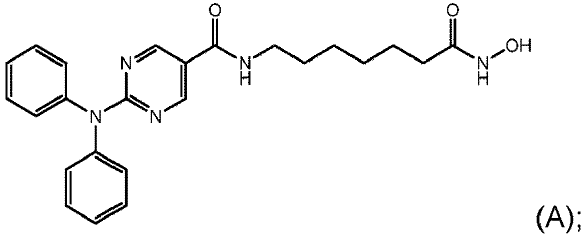 oder Verbindung B 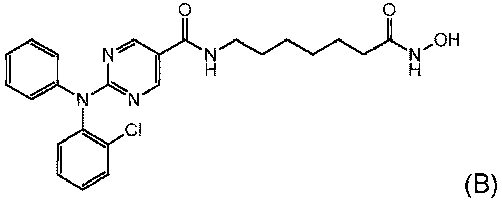 handelt.

2. Verfahren nach Anspruch 1, wobei es sich bei der Probe um eine Myelomprobe oder eine Knochenmarkprobe handelt.

3. Verfahren nach Anspruch 1, wobei Schritt c) das Feststellen umfasst, das die HDAC6-Behandlung wirksam ist, wenn die HDAC6-Biomarker-RNA, bestehend aus der Nukleinsäuresequenz SEQ ID NR:26, um das Zweifache oder mehr herunterreguliert ist.

4. Verfahren nach einem der Ansprüche 1 - 3, wobei es sich bei dem HDAC6-Inhibitor um Verbindung A 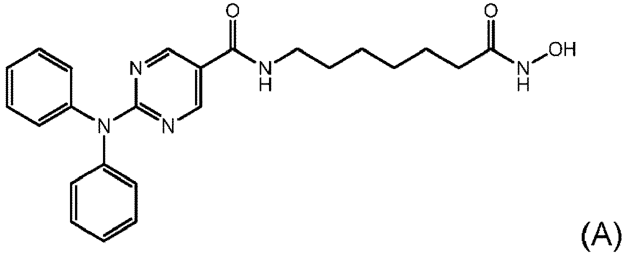 handelt.

5. Verfahren nach einem der Ansprüche 1 - 3, wobei es sich bei dem HDAC6-Inhibitor um Verbindung B 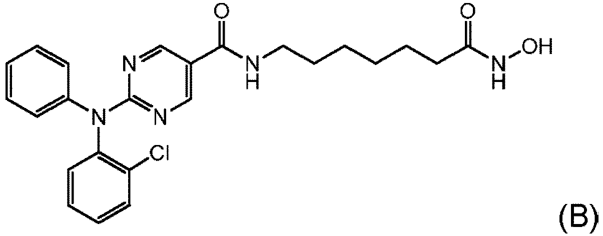 handelt.

6. HDAC6-Inhibitor zur Verwendung bei der Behandlung eines Individuums, wobei die Behandlungseffizienz des HDAC6-Inhibitors bei dem Individuum unter Verwendung des Verfahrens nach Anspruch 1 überwacht wird, wobei die Behandlung ferner eine zusätzlich Menge des HDAC6-Inhibitors umfasst, wenn das Verfahren nach Anspruch 1 in Schritt c) bestimmt, dass die HDAC6-Behandlung nicht wirksam ist,
wobei es sich bei dem HDAC6-Inhibitor um Verbindung A 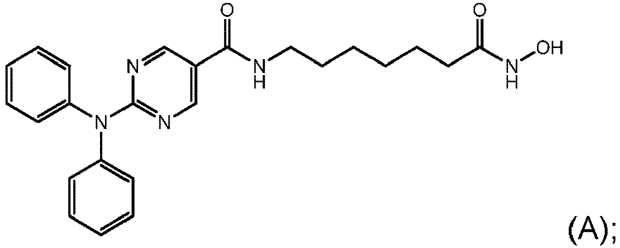 oder Verbindung B 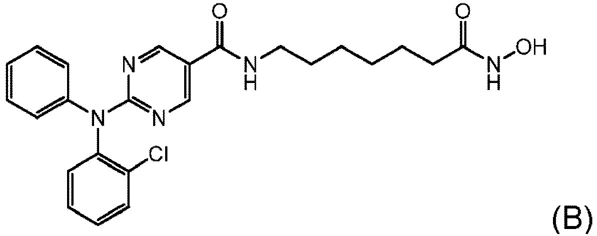 handelt.

## Revendications

1. Procédé pour la surveillance de l'efficacité de traitement d'un inhibiteur d'histone désacétylase 6 (HDAC6) chez un sujet comprenant :
a) la mise à disposition d'un échantillon biologique ayant été pris d'un sujet qui a reçu une quantité thérapeutiquement efficace d'un inhibiteur de HDAC6 ;
b) la détermination de la quantité de l'ARN (acide ribonucléique) biomarqueur de HDAC6 constitué de la séquence d'acides nucléiques de la SEQ ID NO: 26 dans l'échantillon ; et
c) la conclusion du fait que le traitement par HDAC6 est efficace si l'ARN biomarqueur de HDAC6 constitué de la séquence d'acides nucléiques de la SEQ ID NO: 26 est régulé à la baisse ;
l'inhibiteur de HDAC6 étant le composé A 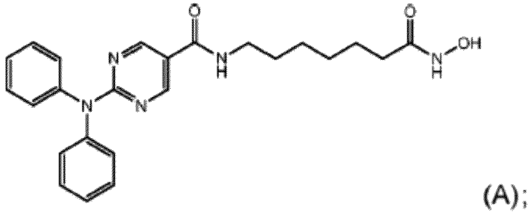 ou le composé B

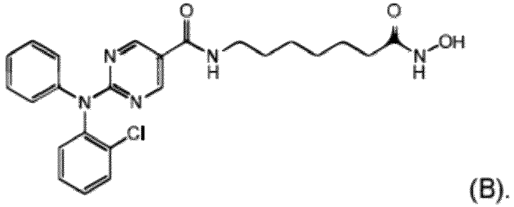

2. Procédé selon la revendication 1, l'échantillon étant un échantillon de myélome, ou un échantillon de moelle osseuse.

3. Procédé selon la revendication 1, l'étape c) comprenant la conclusion du fait que le traitement par HDAC6 est efficace si l'ARN biomarqueur de HDAC6 constitué de la séquence d'acides nucléiques de la SEQ ID NO: 26 est régulé à la baisse 2 fois ou plus.

4. Procédé selon l'une quelconque des revendications 1 à 3, l'inhibiteur de HDAC6 étant le composé A

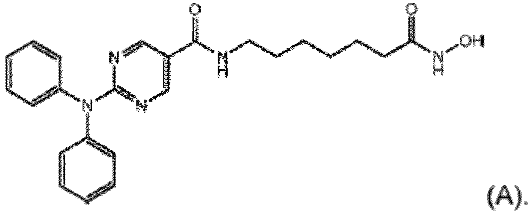

5. Procédé selon l'une quelconque des revendications 1 à 3, l'inhibiteur de HDAC6 étant le composé B

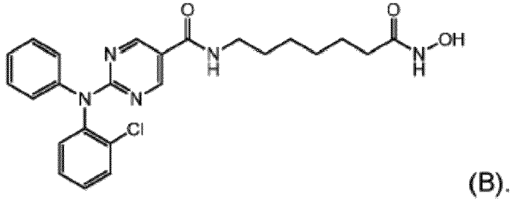

6. Inhibiteur de HDAC6 pour une utilisation dans le traitement d'un sujet, l'efficacité de traitement de l'inhibiteur de HDAC6 chez le sujet étant surveillée en utilisant le procédé selon la revendication 1, le traitement comprenant en outre une quantité supplémentaire de l'inhibiteur de HDAC6 si le procédé selon la revendication 1 a déterminé dans l'étape c) que le traitement par HDAC6 n'est pas efficace, l'inhibiteur de HDAC6 étant le composé A 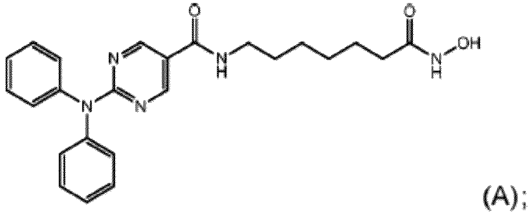 ou le composé B
